Europäisches Patentamt

European Patent Office

Office européen des brevets

(11) Publication number : **0 433 744 B1**

# EUROPEAN PATENT SPECIFICATION

(45) Date of publication of patent specification :
25.08.93 Bulletin 93/34

(51) Int. Cl.⁵ : **A61K 35/74,** A61K 39/05,
A61K 45/05

(21) Application number : **90123092.0**

(22) Date of filing : **03.12.90**

(54) **Semisynthetic derivatives with immunomodulation activity, suitable for parenteral and oral administration.**

(30) Priority : **14.12.89 IT 2269389**

(43) Date of publication of application :
26.06.91 Bulletin 91/26

(45) Publication of the grant of the patent :
25.08.93 Bulletin 93/34

(84) Designated Contracting States :
**AT BE CH DE DK ES FR GB GR LI LU NL SE**

(56) References cited :
EP-A- 0 013 651
FR-A- 1 320 769
FR-A- 2 269 961
FR-A- 2 561 523

(56) References cited :
MEDECINE ET MALADIES INFECTIEUSES,
vol. 8, no. 9, pages 408-414; B. BIZZINI et al.:
"Isolement et caractérisation d'une fraction,
dite fraction P40 à partir de Corynebacterium
granulosum"
CHEMICAL ABSTRACTS, vol. 95, no. 3, 20th
July 1981, abstract no. 18406h, Columbus,
Ohio, US; R.B. HERBERMAN et al.:
"Augmentation of natural killer (NK) cell
activity by interferon and interferon inducers"

(73) Proprietor : **LABORATOIRE MEDIDOM S.A.**
**7, Place du Molard**
**CH-Geneve (CH)**

(72) Inventor : **Volpato, Ivo**
**Via C. Cattaneo, 14**
**I-06100 San Mariano (Perugia) (IT)**
Inventor : **Borella, Fabio**
**Via Balzaretti, 36**
**I-20133 Milan (IT)**

(74) Representative : **Gervasi, Gemma et al**
**NOTARBARTOLO & GERVASI Srl Viale Bianca Maria 33**
**I-20122 Milan (IT)**

## Description

It is known that certain microorganisms such as Mycobacterium bovis, Corynebacterium parvum, Corynebacterium granulosum and Listeria monocytogenes are able to stimulate the functionality of the immunocompetent system, whether normal or depressed.

It is also known that said property is determined by macromolecular compounds such as peptidoglycan and/or glycoproteins which constitute the membrane of microorganisms.

Various bacterial fractions have been isolated consisting of macromolecules of similar type with qualitatively overlapping activity, even if of different physico-chemical characteristic such as solubility, or with a different degree of purification.

Of particular interest is the insoluble fraction, known as P 40, obtained from Corynebacterium granulosum (P. Lallouette, B. Bizzini and M. Rynaud (NIG. M. 1975, 25, 13).

This fraction demonstrates a wide range of biological activities: it stimulates the reticuloendothelial system, increases the resistance to bacterial infections in the mouse, facilitates the production of antibodies against soluble and insoluble antigens, and increases the resistance to various experimental tumours in the mouse (B. Bizzini, E.H. Relyveld and E. Henocq, Vaccine, 1985, 3, 153).

This fraction also demonstrates synergic effects on the activity of antiviral drugs (M. Fattal-German and B. Bizzini, Biomed and Pharmacotherap., 1988, 42, 79), potentiates the effects of antiinfluenza vaccination (M. Fattal-German, A. German and B. Bizzini, Biomed and Pharmacotherap., 1988, 42, 73), induces interferone production (M. Kita, M. Yamajii, E.H. Relyverd, B. Bizzini and T. Kishide in Advances in Immunomodulation, Eds. B. Bizzini and E. Bonmassar, 1988, 153, Pythagora Press (Rome).

Notwithstanding this very important activity, said fraction has demonstrated activity only via parenteral administration.

However, in therapy involving drugs of immunomodulation activity, the availability of a pharmaceutical form for oral administration is of basic importance in that said therapy is directed towards recurring degenerative pathological events which require long periods of administration, and is used in infants and children.

We have now discovered semisynthetic derivatives of immunomodulators of bacterial origin which can be administered both parenterally and orally, to provide high immunomodulation activity. Said semisynthetic derivatives are obtained by conjugating the fraction BV 88, isolated from Corynebacterium granulosum IV-86 ATCC strain 53966, with amino acids or polyamino acids after activating said fraction with glutaraldehyde or conjugating with carbodiimide.

The characteristics and advantages of the semisynthetic derivatives of immunomodulators of bacterial origin according to the present invention and the process for their preparation are further illustrated in the course of the detailed description given hereinafter.

Said derivatives are prepared by the following stages:

a) A strain of Corynebacterium granulosum IV-86 containing fractions with immunomodulation activity, filed under ATCC No. 53966, is isolated from pathological material;

b) An insoluble fraction which demonstrates immunomodulation and antitumoral activity when administered parenterally and known as BV88 is isolated from said strain;

c) The BV 88 fraction is conjugated with amino acids or with polyamino acids after activating with glutaraldehyde or conjugating with carbodiimide.

In this manner semisynthetic derivatives with immunomodulation properties are obtained which are suitable for both parenteral and oral administration.

The strain Corynebacterium granulosum IV-86, ATCC 53966 has the following characteristics:

anaerobic rods, non-sporogenic, Gram-positive;

rapid growth in usual culture media; the addition of serum to the medium results in more abundant growth;

produces smooth colonies on agar-blood plates;

does not produce indole;

does not reduce nitrates

does not generate $H_2S$;

produces catalysis;

ferments glucose but not mannitol or lactose;

produces non-specific stimulation of the reticuloendothelial system.

The BV 88 fraction is characterised chemically by the following methods:

- Analysis of the enzymatic degradation of the fraction:

The BV 88 fraction is digested by 1% trypsin or chymotrypsin in a 0.1 M $NH_4CO_3H$ solution (pH 8).

- Test of solubilization by chemical action:

The solubilization test is conducted with a mixture of 0.1N NaOH/9% formic acid/6M guanidine and Triton X-100.
- Determination of amino acid composition:
The analysis was conducted on acid hydrolysate using an amino acid analyzer.

Methionine and cysteine were determined by performic oxidation (S. Moore, J. Biol. Chem., 1963, 238, 235)

Diaminopimelic acid (DMP) was determined by automatic analysis by subtracting methionine from the DAP + methionine content.

Muramic acid was determined by the Spackman system (Anal. Chem., 1958, 30, 1190).

Tryptophan was determined by the colorimetric method of Gaitonde and Dovey (Biochem. J., 1970, 117, 907).
- Determination and identification of glucides:
The neutral sugars were determined by the anthrone method (P. Albesheim and coll., Carbohydrate Res. 1967, 5, 340) and the hexosamines by the method of Elson and Morgan (Biochem. J., 1933, 27, 1824).

The results of the chemical analyses on the BV 88 fraction are shown in Table 1.

TABLE 1

<u>Chemical composition of BV 88 fraction</u>

g/100 g (dry weight)

Amino acid

| | |
|---|---|
| HIS | 1.30-1.85 |
| LYS | 2.89-3.31 |
| ARG | 2.95-3.15 |
| ASP | 3.0 -4.05 |
| THR | 1.75-2.15 |
| SER | 1.11-1.49 |
| GLU | 5.94-6.51 |
| PRO | 0.89-1.16 |
| ALA | 3.04-3.52 |
| GLY | 3.02-3.47 |
| CYS | 0.27-0.34 |
| VAL | 2.64-3.03 |
| MET | 0.96-1.27 |
| ILEU | 1.52-1.97 |
| LEU | 2.95-3.14 |
| TYR | 0.88-0.96 |
| PHE | 2.0 -2.30 |
| TRY | 1.0 -1.17 |

| | |
|---|---|
| Diaminopimelic acid | 2.51-3.74 |
| Neutral sugars | 12.12-14.21 |
| Hexosamines | 9.32-10.44 |
| Muramic acid | 8.92-11.74 |
| Ash | 0.5 -1 |

With regard to biological activity of the BV 88 fraction, an evaluation of the activity of the reticuloendothelial system was effected.

This test was carried out on 16-18 g Swiss mice using the method described by Halpern and coll. (Ann. Inst. Pasteur, 1951, 80, 582; J. Reticuloendoth. Soc., 1964, 1, 77), the phagocytic index $K/K_o$ being calculated by the method of Halpern and coll. (Ann. Inst. Pasteur, 1951, 80, 582).

The substance was injected on day 0 and elimination of the colloidal carbon particles was monitored for six days after administration.

The results are given in Table 2.

TABLE 2

Effect of BV 88 fraction on the phagocytic index $K/K_o$

| Treatment | Dose | $K/K_o$ |
|---|---|---|
| Controls | - | 1 |
| BV 88 | 250 µg/mouse (determined on 3rd day) | 1.48 |
| BV 88 | 250 µg/mouse (determined on 6th day) | 2.94 |

To prepare the semisynthetic derivatives according to the present invention a suitably activated suspension of the BV 88 fraction is reacted for conjugation with an amino acid or polyamino acid.

The BV 88 fraction can be activated by treatment with a CHO-$(CH_2)_n$-CHO dialdehyde where n is between 2 and 4 in a stage preceding the conjugation reaction, or by treatment with carbodiimide in the BV 88 and amino acid mixture prepared for the conjugation reaction. The dialdehyde used can for example be glutaraldehyde.

If the BV 88 fraction is activated with a dialdehyde, a suspension of BV 88 in pH 7.4 isotonic phosphate buffer (PBS) is prepared having a BV 88 content of between 1 and 20 mg/ml, and a solution of dialdehyde in the same buffer is added.

The activation reaction is conducted at as temperature of between 4 and 37°C for a time of between 15 minutes and 4 hours.

Alternatively the activation of BV 88 with dialdehyde can be effected by treating said BV 88 suspension with a polyphenylalanine-polylysine solution prior to the aforesaid operations.

The BV 88 fraction activated with dialdehyde is used for the conjugation reaction with diaminomonocarboxylic amino acids or with polyamino acids.

This reaction is conducted by treating a suspension of activated BV 88 in PBS containing between 1 and 20 mg/ml of BV 88 with an amino acid solution having a concentration of between 1 and 20 mg/ml or a polyamino acid in powder form, then raising the pH to 9.5 with a 0.5 M $NaHCO_3/Na_2CO_3$, reacting for 1-3 hours at a temperature of between 4 and 37°C and then for 12 hours at a temperature of less than 10°C.

In this manner the semisynthetic derivative is obtained, in which the weight ratio of the amino acid to the BV 88 fraction is between 0.05:1 and 1.5:1 or the weight ratio of the polyamino acid to the BV 88 fraction is between 0.001:1 and 0.05:1.

The amino acids to be used in the described process are lysine, arginine, glutamine and asparagine, and the polyamino acids are polylysine, polyarginine and polylysine-arginine, of molecular weight between 1000 and 15,000 daltons.

If the BV 88 fraction is activated by carbodiimide conjugation, a 0.1-10 mg/ml solution of an amino acid in pH 5 0.1 M sodium acetate buffer is added to a 1-20 mg/ml suspension of the BV 88 fraction in the same buffer, and a carbodiimide solution is added to the mixture obtained. The amino acid conjugation reaction is conducted at ambient temperature while maintaining pH 5 by correction with 0.1 N HCl.

In this case the weight ratio of the amino acid to the BV 88 fraction is between 0.05:1 and 0.5:1. The amino acids to be used with this type of activation are the monoaminomonocarboxylic and monoaminodicarboxylic acids such as leucine and aspartic acid.

The process for preparing the semisynthetic derivatives of the BV 88 fraction according to the present invention is illustrated by the following examples.

EXAMPLE 1

Preparation of the derivative 1/BV

10 mg of BV 88 are suspended in 2 ml of PBS buffer. 2 ml of a 2.5% glutaraldehyde solution in the same buffer are added and the mixture stirred for 2 hours at 37°C.

The thus activated BV 88 fraction (BV 88-Gluta) is collected by centrifuge and washed three times by centrifuging in PBS, after which a suspension of BV 88-Gluta in PBS at a concentration of 10 mg/ml of BV 88 is prepared (suspension A).

Separately, a solution of L-lysine hydrochloride in physiological solution having an L-lysine concentration of 10 mg/ml is prepared and the pH adjusted to 7 with 1N NaOH (Solution B).

The conjugation reaction is conducted by mixing 1 volume of solution B with 1 volume of solution A, adding 0.1 volumes of 0.5 M $NaHCO_3$/$Na_2CO_3$ (pH 9.5) buffer and leaving the mixture to react for 2 hours at 37°C while stirring and then for 12 hours at 4°C.

After reaction, the derivative is sedimented by centrifuging and washed twice by resuspension in physiological solution and centrifuging.

The product obtained in this manner is resuspended in physiological solution to a concentration of 10 mg/ml and then lyophilized.

In this manner the semisynthetic derivative of the BV 88 fraction with L-lysine, coded 1/BV, is obtained with a weight ratio of amino acid to BV 88 fraction of 1:1. The yield is quantitative.

EXAMPLES 2 to 4

Preparation of the derivatives 2/BV, 3/BV and 4/BV

These examples are conducted following the procedure of Example 1 but with the difference that 0.5, 0.25 and 0.125 volumes of solution B are added to 1 volume of solution A respectively, the total volume being made up with physiological solution.

In this manner the semisynthetic derivatives of the BV 88 fraction with L-lysine, coded 2/BV, 3/BV and 4/BV, are obtained with a weight ratio of amino acid to BV 88 fraction of 0.5:1, 0.25:1 and 0.125:1 respectively. The yield is quantitative.

EXAMPLE 5

Preparation of the derivative 5/BV

0.1 volumes of 0.5 M $NaHCO_3$/$Na_2CO_3$ (pH 9.5) buffer are added to 1 volume of suspension A obtained as described in Example 1, and 20 mg of poly-L-lysine in the hydrobromide powder form of M.W. 1000-4000 are added per gram of BV 88 fraction.

The mixture is left to react for 2 hours at 37°C while stirring and then for 12 hours at 4°C.

The product obtained is washed twice by centrifuging in physiological solution and the sediment is resuspended in physiological solution to a concentration of 10 mg/ml and then lyophilized.

In this manner the semisynthetic derivative of the BV 88 fraction with poly-L-lysine, coded 5/BV, is obtained with a weight ratio of amino acid to BV 88 fraction of 0.02:1.

EXAMPLES 6 and 7

Preparation of the derivatives 6/BV and 7/BV

These examples are conducted following the procedure of Example 5 but with the difference that 5 and 2 mg of the poly-L-lysine are added to suspension A per gram of BV 88 fraction respectively.

In this manner the semisynthetic derivatives of the BV 88 fraction with poly-L-lysine, coded 6/BV and 7/BV, are obtained with a weight ratio of amino acid to BV 88 fraction of 0.005:1 and 0.002:1 respectively.

EXAMPLE 8

Preparation of the derivative 8/BV

A 0.5 mg/ml solution of L-leucine in 0.1 M sodium acetate buffer (pH 5) is prepared.

A 10 mg/ml suspension of the BV 88 fraction is prepared separately in the same buffer, and 0.5 ml of this suspension are mixed with 2 ml of the L-leucine solution.

2 mg of 1-ethyl-3(3-dimethylaminopropyl)-carbodiimide dissolved in 0.5 ml of distilled water are added to the mixture, which is then kept under continuous stirring for 4 hours, constantly adjusting the pH to 5 by adding 0.1 N HCl at ambient temperature.

After the reaction, the mixture is centrifuged and the sediment washed twice by resuspending in physiological solution and centrifuging.

The precipitate is finally taken up in physiological solution at a concentration of 10 mg/ml and lyophilized.

In this manner the semisynthetic derivative of the BV 88 fraction with L-leucine, coded 8/BV, is obtained with a weight ratio of amino acid to BV 88 fraction of 1:5.

EXAMPLE 9

Preparation of the derivative 9/BV

For this example the procedure described in example 8 is followed, using as amino acid L-aspartic acid in a weight ratio to the BV 88 fraction of 1:10.

In this manner the relative semisynthetic derivative coded 9/BV is obtained.

Experimental pharmacological part

The semisynthetic derivatives obtained in the preceding examples were studied in a trial to determine their immunomodulation and antitumoral activity after oral and parenteral administration compared with the initial BV 88 fraction.

The method of operation and the results of the trial are given under the following headings.

A) Capacity of fraction BV 88 derivatives to stimulate the function of the reticuloendothelial system

This test was conducted by the method of Halpern and coll. (Ann. Inst. Pasteur 1951, 80, 582).

For analysis of the effects following parenteral (intravenous) administration, the substances were administered in a single dose of 500 $\mu$g/mouse at time 0, whereas for analysis of the effects following oral administration, they were administered by gastric probe for 6 days at a dose of 1 mg/mouse/day.

The examination of the functionality of the reticuloendothelial system ($K/K_o$ index) was made 6 days following treatment.

The results are given in Table 3.

## TABLE 3

### Effect of semisynthetic fraction BV 88 derivatives on the activity of the reticuloendothelial system ($K/K_o$ index)

| Compound | Method of administration | Dose | $K/K_o$ |
|---|---|---|---|
| control | - | - | 1 |
| BV 88 | iv | 500 µg/mouse | 4.06 |
|  | oral | 1 mg/mouse for 6 days | 1.37 |
| 1/BV | iv | 500 µg/mouse | 3.96 |
|  | oral | 1 mg/mouse for 6 days | 1.50 |
| 2/BV | iv | 500 µg/mouse | 3.73 |
|  | oral | 1 mg/mouse for 6 days | 1.66 |
| 3/BV | iv | 500 µg/mouse | 3.82 |
|  | oral | 1 mg/mouse for 6 days | 1.90 |
| 4/BV | iv | 500 µg/mouse | 3.97 |
|  | oral | 1 mg/mouse for 6 days | 2.05 |
| 5/BV | iv | 500 µg/mouse | 3.6 |
|  | oral | 1 mg/mouse for 6 days | 1.49 |
| 6/BV | iv | 500 µg/mouse | 3.88 |
|  | oral | 1 mg/mouse for 6 days | 1.74 |
| 7/BV | iv | 500 µg/mouse | 4.02 |
|  | oral | 1 mg/mouse for 6 days | 1.97 |
| 8/BV | iv | 500 µg/mouse | 3.90 |
|  | oral | 1 mg/mouse for 6 days | 1.68 |
| 9/BV | iv | 500 µg/mouse | 3.99 |
|  | oral | 1 mg/mouse for 6 days | 1.72 |

B) Effect of fraction BV 88 derivatives on experimental infection by Listeria monocytogenes in the mouse

The experimental infection was induced in the mouse by i.v. administration of $1.2 \times 10^4$ bacteria in 0.2 ml of an 18 hour culture.

The treatment was via gastric probe, administering 1 mg of the various derivatives per day for three days before the experimental infection.

Table 4 shows the percentage protection against death from the infection, obtained by checking the animals 8 days after infection.

## TABLE 4

Effect of fraction BV 88 derivatives on experimental infection in the mouse induced by Listeria monocytogenes

| Compound | treatment | % protection against death |
|----------|-----------|---------------------------|
| BV 88 | 1 mg/oral/3 days | 10% |
| 1/BV | 1 mg/oral/3 days | 30% |
| 2/BV | 1 mg/oral/3 days | 30% |
| 3/BV | 1 mg/oral/3 days | 40% |
| 4/BV | 1 mg/oral/3 days | 45% |
| 5/BV | 1 mg/oral/3 days | 25% |
| 6/BV | 1 mg/oral/3 days | 25% |
| 7/BV | 1 mg/oral/3 days | 40% |
| 8/BV | 1 mg/oral/3 days | 30% |
| 9/BV | 1 mg/oral/3 days | 22% |

The BV 88 fraction administered at a dose of 1 mg i.v. 7 days before infection gives 80% protection; the derivatives of the invention behave analogously on i.v. administration.

C) Effect of fraction BV 88 derivatives on experimental infection by HSV-1 in the mouse

The experimental infection was induced in the mouse by i.v. administration of HSV 1 virus cultivated on VERO cells, in a volume of 0.5 ml of a 1:1000 dilution of a culture containing $3.2 \times 10^8$ DICC$_{50}$.

The treatment was given orally for three days before infection, at a dose of 1 mg/mouse/day. The results are given in Table 5.

## TABLE 5

Effect of treatment with fraction BV 88 derivatives on Experimental infection in the mouse induced by HSV-1

| Compound | treatment | % protection |
|----------|-----------|--------------|
| BV 88 | 1 mg/oral/3 days | 7% |
| 1/BV | 1 mg/oral/3 days | 13% |
| 2/BV | 1 mg/oral/3 days | 27% |
| 3/BV | 1 mg/oral/3 days | 34% |
| 4/BV | 1 mg/oral/3 days | 47% |
| 5/BV | 1 mg/oral/3 days | 33% |
| 6/BV | 1 mg/oral/3 days | 34% |
| 7/BV | 1 mg/oral/3 days | 40% |
| 8/BV | 1 mg/oral/3 days | 13% |
| 9/BV | 1 mg/oral/3 days | 20% |

D) Effect of fraction BV 88 derivatives on splenic NK cells in the mouse

The mice were treated for 5 days orally with 1 mg of derivative in 0.5 ml of water.
10 days after treatment the natural killer activity (NK cells) was determined using splenocytes originating from the treated animals as effecting cells and YAC-1 tumoral cells tagged with Cr 51 as target.
The activity was measured at two different ratios of effecting cells to target cells (50:1 and 100:1).
The results are given in Table 6.

TABLE 6

Effect of fraction BV 88 derivatives on the activity of splenic NK cells in the mouse

| Compound | treatment/mouse | specific cytotoxicity % | |
|---|---|---|---|
| | | 50:1 | 100:1 |
| Control | - | 13.4 | 17.8 |
| BV 88 | 1 mg/oral/5 days | 16.5 | 19.2 |
| 1/BV | 1 mg/oral/5 days | 17.3 | 20.8 |
| 2/BV | 1 mg/oral/5 days | 17.0 | 19.2 |
| 3/BV | 1 mg/oral/5 days | 17.6 | 24.9 |
| 4/BV | 1 mg/oral/5 days | 20.2 | 27.8 |
| 5/BV | 1 mg/oral/5 days | 11.3 | 15.0 |
| 6/BV | 1 mg/oral/5 days | 13.5 | 19.2 |
| 7/BV | 1 mg/oral/5 days | 20.4 | 30.8 |
| 8/BV | 1 mg/oral/5 days | 14.5 | 19.0 |
| 9/BV | 1 mg/oral/5 days | 14.5 | 21.6 |

E) Effect of BV 88 derivatives on growth of the Lewis solid tumour induced in the C 57 BL/6 mouse

The tumour was induced by i.m. injection of $10^4$ tumoral cells.

The treatment was conducted by orally administering 1 mg of derivative in 0.5 ml of water every 2 days for 10 days.

The survival times compared with the controls are shown in Table 7.

TABLE 7

Effect of BV 88 derivatives on growth of the Lewis solid tumour

| Compound | treatment | Time to attain 100% mortality |
|---|---|---|
| Control | - | 36 days |
| BV 88 | 1mg/oral/10 days on alternate days | 38 " |
| 1/BV | " | 39 " |
| 2/BV | " | 38 " |
| 3/BV | " | 40 " |
| 4/BV | " | 38 " |
| 5/BV | " | 45 " |
| 6/BV | " | 43 " |
| 7/BV | " | 43 " |
| 8/BV | " | 37 " |
| 9/BV | " | 37 " |

By virtue of the characteristics determined from the aforegiven trials, the derivatives of the present invention can be used for preparing pharmaceutical compounds with immunomodulation and antitumoral activity for parenteral and oral administration.

**Claims**

**Claims for the following Contracting States : AT, BE, CH, DE, DK, FR, GB, LI, LU, NL, SE**

1.  Semisynthetic derivatives with immunomodulation activity for parenteral or oral administration, consisting of conjugates of amino acids or polyamino acids with an insoluble fraction coded BV 88, which is isolated from Corynebacterium granulosum IV-86 ATCC strain 53966 and consists of peptidoglycan and glycoproteins.

2.  Semisynthetic derivatives as claimed in claim 1, characterised in that said amino acids are diaminomonocarboxylic, monoaminomonocarboxylic and monoaminodicarboxylic amino acids.

3.  Semisynthetic derivatives as claimed in claim 1, characterised in that said amino acids are lysine, arginine, glutamine, asparagine, leucine and aspartic acid.

4.  Semisynthetic derivatives as claimed in claim 1, characterised in that said polyamino acids are polylysine, polyarginine and polylysine-arginine, of molecular weight 1000-15,000.

5.  Semisynthetic derivatives as claimed in claim 1, characterised in that the weight ratio of the contained amino acid to the BV 88 fraction is between 0.05:1 and 1.5:1.

12

6. Semisynthetic derivatives as claimed in claim 1, characterised in that the weight ratio of the contained polyamino acid to the BV 88 fraction is between 0.001:1 and 0.05:1.

7. A process for preparing semisynthetic derivatives with immunomodulation activity for parenteral or oral administration, consisting of conjugates of amino acids or polyamino acids with an insoluble fraction coded BV 88, which is isolated from Corynebacterium granulosum IV-86 ATCC strain 53966 and consists of peptidoglycan and glycoproteins, characterised in that a suspension of the BV 88 fraction activated with a CHO-$(CH_2)_n$-CHO dialdehyde where n is between 2 and 4 or conjugated with carbodiimide is reacted with an amino acid or polyamino acid for conjugation.

8. A process as claimed in claim 7, characterised in that the activation with dialdehyde is effected by treating a 1-20 mg/ml suspension of BV 88 in pH 7.4 buffer solution with a solution of dialdehyde in the same buffer.

9. A process as claimed in claim 7, characterised in that the reaction for conjugating the BV 88 fraction if activated with dialdehyde is effected by treating a suspension of activated BV 88 containing between 1 and 20 mg/ml of BV 88 with an amino acid solution having a concentration of between 1 and 20 mg/ml or with a polyamino acid in powder form at pH to 9.5 at a temperature of between 4 and 37°C.

10. A process as claimed in claim 7, characterised in that the amino acids used for conjugation with BV 88 if activated with dialdehyde are diaminomonocarboxylic amino acids.

11. A process as claimed in claim 7, characterised in that the polyamino acids used for conjugation with BV 88 if activated with dialdehyde have a molecular weight of between 1000 and 15,000 daltons.

12. A process as claimed in claim 7, characterised in that said dialdehyde is glutaraldehyde.

13. A process as claimed in claim 7, characterised in that if the BV 88 fraction is conjugated with carbodiimide, a 0.1-10 mg/ml solution of an amino acid in pH 5 buffer is added to a 1-20 mg/ml suspension of the BV 88 fraction in the same buffer, and a carbodiimide solution is added to the mixture obtained.

14. A process as claimed in claim 7, characterised in that if the BV 88 fraction is conjugated with carbodiimide, the amino acid conjugation reaction is conducted at pH 5 at ambient temperature.

15. A process as claimed in claim 7, characterised in that the amino acids used for conjugation with BV 88 and carbodiimide are monoaminomonocarboxylic and monoaminodicarboxylic amino acids.

16. The use of semisynthetic derivatives consisting of conjugates of amino acids or polyamino acids with an insoluble fraction coded BV 88, which is isolated from Corynebacterium granulosum IV-86 ATCC strain 53966 and consists of peptidoglycan and glycoproteins, in the preparation of pharmaceutical compositions with immunomodulation and antitumoral activity for parenteral and oral administration.

**Claims for the following Contracting States : GR, ES**

1. A process for preparing semisynthetic derivatives with immunomodulation activity for parenteral or oral administration, consisting of conjugates of amino acids or polyamino acids with an insoluble fraction coded BV 88, which is isolated from Corynebacterium granulosum IV-86 ATCC strain 53966 and consists of Peptidoglycan and glycoproteins, characterised in that a suspension of the BV 88 fraction activated with a CHO-$(CH_2)_n$-CHO dialdehyde where n is between 2 and 4 or conjugated with carbodiimide is reacted with an amino acid or polyamino acid for conjugation.

2. A process as claimed in claim 1, characterised in that the activation with dialdehyde is effected by treating a 1-20 mg/ml suspension of BV 88 in pH 7.4 buffer solution with a solution of dialdehyde in the same buffer.

3. A process as claimed in claim 1, characterised in that the reaction for conjugating the BV 88 fraction if activated with dialdehyde is effected by treating a suspension of activated BV 88 containing between 1 and 20 mg/ml of BV 88 with an amino acid solution having a concentration of between 1 and 20 mg/ml or with a polyamino acid in powder form at pH to 9.5 at a temperature of between 4 and 37°C.

4. A process as claimed in claim 1, characterised in that the amino acids used for conjugation with BV 88 if activated with dialdehyde are diaminomonocarboxylic amino acids.

5. A process as claimed in claim 1, characterised in that the polyamino acids used for conjugation with BV 88 if activated with dialdehyde have a molecular weight of between 1000 and 15,000 daltons.

6. A process as claimed in claim 1, characterised in that said dialdehyde is glutaraldehyde.

7. A process as claimed in claim 1, characterised in that if the BV 88 fraction is conjugated with carbodiimide, a 0.1-10 mg/ml solution of an amino acid in pH 5 buffer is added to a 1-20 mg/ml suspension of the BV 88 fraction in the same buffer, and a carbodiimide solution is added to the mixture obtained.

8. A process as claimed in claim 1, characterised in that if the BV 88 fraction is conjugated with carbodiimide, the amino acid conjugation reaction is conducted at pH 5 at ambient temperature.

9. A process as claimed in claim 1, characterised in that the amino acids used for conjugation with BV 88 and carbodiimide are monoaminomonocarboxylic and monoaminodicarboxylic amino acids.

**Patentansprüche**

**Patentansprüche für folgende Vertragsstaaten : AT, BE, CH, DE, DK, FR, GB, LI, LU, NL, SE**

1. Halbsynthetische Derivate mit immunmodulierender Aktivität zur parenteralen oder oralen Verabreichung, bestehend aus Konjugaten von Aminosäuren oder Polyaminosäuren mit einer unlöslichen Fraktion bezeichnet als BV 88, welche aus Corynebacterium granulosum IV-86 ATCC Stamm 53966 isoliert ist und aus Peptidoglycan und Glycoproteinen besteht.

2. Halbsynthetische Derivate nach Anspruch 1, dadurch gekennzeichnet, daß die genannten Aminosäuren Diaminomonocarbonsäuren, Monoaminomonocarbonsäuren und Monoaminodicarbonsäuren sind.

3. Halbsynthetische Derivate nach Anspruch 1, dadurch gekennzeichnet, daß die genannten Aminosäuren Lysin, Arginin, Glutamin, Asparagin, Leucin und Asparaginsäure sind.

4. Halbsynthetische Derivate nach Anspruch 1, dadurch gekennzeichnet, daß die genannten Polyaminosäuren Polylysin, Polyarginin und Polylysin-Arginin mit einem Molekulargewicht von 1.000 bis 15.000 sind.

5. Halbsynthetische Derivate nach Anspruch 1, dadurch gekennzeichnet, daß das Gewichtsverhältnis der enthaltenen Aminosäure zur BV 88-Fraktion zwischen 0,05 : 1 und 1,5 : 1 beträgt.

6. Halbsynthetische Derivate nach Anspruch 1, dadurch gekennzeichnet, daß das Gewichtsverhältnis der enthaltenen Polyaminosäure zur BV 88-Fraktion zwischen 0,001 : 1 und 0,005 : 1 beträgt.

7. Verfahren zur Herstellung halbsynthetischer Derivate mit immunmodulierender Aktivität zur parenteralen oder oralen Verabreichung, bestehend aus Konjugaten von Aminosäuren oder Polyaminosäuren mit einer unlöslichen Fraktion bezeichnet als BV 88, welche aus Corynebacterium granulosum IV-86 ATCC Stamm 53966 isoliert ist und aus Peptidoglycan und Glycoproteinen besteht, dadurch gekennzeichnet, daß man zur Konjugation eine Suspension der BV 88-Fraktion, welche mit einem $CHO\text{-}(CH_2)_n\text{-}CHO$-Dialdehyd, worin n zwischen 2 und 4 liegt, aktiviert ist oder mit Carbodiimid konjugiert ist, mit einer Aminosäure oder Polyaminosäure umsetzt.

8. Verfahren nach Anspruch 7, dadurch gekennzeichnet, daß die Aktivierung mit Dialdehyd erzielt wird durch Behandlung einer 1 - 20 mg/ml Suspension von BV 88 in einer Pufferlösung bei pH 7,4 mit einer Lösung von Dialdehyd in dem gleichen Puffer.

9. Verfahren nach Anspruch 7, dadurch gekennzeichnet, daß die Reaktion zur Konjugation der BV 88-Fraktion im Falle der Aktivierung mit Dialdehyd durch Behandlung einer Suspension von aktiviertem BV 88, welche zwischen 1 und 20 mg/ml von BV 88 enthält, mit einer Aminosäurelösung, welche eine Konzentration zwischen 1 und 20 mg/ml hat oder mit einer pulverförmigen Polyaminosäure bei einem pH-Wert bis zu 9,5 und einer Temperatur zwischen 4 und 37 °C durchgeführt wird.

10. Verfahren nach Anspruch 7, dadurch gekennzeichnet, daß die Aminosäuren, welche für die Konjugation von BV 88 im Falle der Aktivierung mit Dialdehyd verwendet werden, Diaminomonocarbonsäuren sind.

11. Verfahren nach Anspruch 7, dadurch gekennzeichnet, daß die Polyaminosäuren, welche für die Konjugation von BV 88 im Falle der Aktivierung mit Dialdehyd verwendet werden, ein Molekulargewicht zwischen 1.000 und 15.000 Dalton aufweisen.

12. Verfahren nach Anspruch 7, dadurch gekennzeichnet, daß der genannte Dialdehyd Glutaraldehyd ist.

13. Verfahren nach Anspruch 7, dadurch gekennzeichnet, daß im Falle der Konjugation der BV 88-Fraktion mit Carbodiimid eine 0,1 - 10 mg/ml Lösung einer Aminosäure in einem Puffer bei pH 5 zu einer 1 - 20 mg/ml Suspension von BV 88-Fraktion in dem gleichen Puffer hinzugefügt wird, und der so erhaltenen Mischung eine Carbodiimid-Lösung zugefügt wird.

14. Verfahren nach Anspruch 7, dadurch gekennzeichnet, daß im Falle der Konjugation der BV 88-Fraktion mit Carbodiimid, die Aminosäurekonjugationsreaktion bei pH 5 und Umgebungstemperatur durchgeführt wird.

15. Verfahren nach Anspruch 7, dadurch gekennzeichnet, daß die für die Konjugation mit BV 88 und Carbodiimid verwendeten Aminosäuren Monoaminomonocarbonsäuren und Monoaminodicarbonsäuren sind.

16. Verwendung von halbsynthetischen Derivaten bestehend aus Konjugaten von Aminosäuren oder Polyaminosäuren mit einer unlöslichen Fraktion bezeichnet als BV 88, welche aus Corynebacterium granulosum IV-86 ATCC Stamm 53966 isoliert und aus Peptidoglycan und Glycoproteinen besteht, zur Herstellung von pharmazeutischen Zusammensetzungen mit immunmodulierender und antitumoraler Aktivität zur parenteralen und oralen Verabfolgung.

**Patentansprüche für folgende Vertragsstaaten : GR, ES**

1. Verfahren zur Herstellung halbsynthetischer Derivate mit immunmodulierender Aktivität zur parenteralen oder oralen Verabreichung, bestehend aus Konjugaten von Aminosäuren oder Polyaminosäuren mit einer unlöslichen Fraktion bezeichnet als BV 88, welche aus Corynebacterium granulosum IV-86 ATCC Stamm 53966 isoliert ist und aus Peptidoglycan und Glycoproteinen besteht, dadurch gekennzeichnet, daß man zur Konjugation eine Suspension der BV 88-Fraktion welche mit einem $CHO-(CH_2)_n-CHO$-Dialdehyd, worin n zwischen 2 und 4 liegt, aktiviert ist oder mit Carbodiimid konjugiert ist, mit einer Aminosäure oder Polyaminosäure umsetzt.

2. Verfahren nach Anspruch 1, dadurch gekennzeichnet, daß die Aktivierung mit Dialdehyd erzielt wird durch Behandlung einer 1 - 20 mg/ml Suspension von BV 88 in einer Pufferlösung bei pH 7,4 mit einer Lösung von Dialdehyd in dem gleichen Puffer.

3. Verfahren nach Anspruch 1, dadurch gekennzeichnet, daß die Reaktion zur Konjugation der BV 88-Fraktion im Falle der Aktivierung mit Dialdehyd durch Behandlung einer Suspension von aktiviertem BV 88, welche zwischen 1 und 20 mg/ml von BV 88 enthält, mit einer Aminosäurelösung, welche eine Konzentration zwischen 1 und 20 mg/ml hat oder mit einer pulverförmigen Polyaminosäure bei einem pH bis zu 9,5 und einer Temperatur zwischen 4 und 37 °C durchgeführt wird.

4. Verfahren nach Anspruch 1, dadurch gekennzeichnet, daß die Aminosäuren, welche für die Konjugation von BV 88 im Falle der Aktivierung mit Dialdehyd verwendet werden, Diaminomonocarbonsäuren sind.

5. Verfahren nach Anspruch 1, dadurch gekennzeichnet, daß die Polyaminosäuren, welche für die Konjugation von BV 88 im Falle der Aktivierung mit Dialdehyd verwendet werden, ein Molekulargewicht zwischen 1.000 und 15.000 Dalton aufweisen.

6. Verfahren nach Anspruch 1, dadurch gekennzeichnet, daß der genannte Dialdehyd Glutaraldehyd ist.

7. Verfahren nach Anspruch 1, dadurch gekennzeichnet, daß im Falle der Konjugation der BV 88-Fraktion mit Carbodiimid eine 0,1 - 10 mg/ml Lösung einer Aminosäure in einem Puffer bei pH 5 zu einer 1 - 20 mg/ml Suspension von BV 88-Fraktion in dem gleichen Puffer hinzugefügt wird, und der so erhaltenen Mischung eine Carbodiimid-Lösung zugefügt wird.

8. Verfahren nach Anspruch 1, dadurch gekennzeichnet, daß im Falle der Konjugation der BV 88-Fraktion mit Carbodiimid die Aminosäurekonjugationsreaktion bei pH 5 und Umgebungstemperatur durchgeführt

15

wird.

**9.** Verfahren nach Anspruch 1, dadurch gekennzeichnet, daß die für die Konjugation mit BV 88 und Carbodiimid verwendeten Aminosäuren Monoaminomonocarbonsäuren und Monoaminodicarbonsäuren sind.

## Revendications

### Revendications pour les Etats contractants suivants : AT, BE, CH, DE, DK, FR, GB, LI, LU, NL, SE

**1.** Dérivés semi-synthétiques ayant une activité d'immunomodulation pour administration par voie parentérale ou orale, consistant en des composés conjugués d'acides aminés ou de poly acides aminés avec une fraction insoluble codée BV 88, qui est isolée à partir de la souche IV-86 ATCC 53966 de Corynebacterium granulosum et qui consiste en peptidoglycane et en glycoprotéines.

**2.** Dérivés semi-synthétiques selon la revendication 1, caractérisés en ce que lesdits acides aminés sont des acides diaminomonocarboxyliques, monoaminomonocarboxyliques et monoaminodicarboxyliques.

**3.** Dérivés semi-synthétiques selon la revendication 1, caractérisés en ce que lesdits acides aminés sont la lysine, l'arginine, la glutamine, l'asparagine, la leucine et l'acide aspartique.

**4.** Dérivés semi-synthétiques selon la revendication 1, caractérisés en ce que lesdits polyacides aminés sont la polylysine, la polyarginine et la polylysine-arginine ayant un poids moléculaire de 1000-15 000.

**5.** Dérivés semi-synthétiques selon la revendication 1, caractérisés en ce que le rapport en poids de l'acide aminé qu'ils contiennent à la fraction BV 88 est compris entre 0,05/1 et 1,5/1.

**6.** Dérivés semi-synthétiques selon la revendication 1, caractérisés en ce que le rapport en poids du polyacide aminé qu'ils contiennent à la fraction BV 88 est compris entre 0,001/1 et 0,05/1.

**7.** Procédé de préparation de dérivés semi-synthétiques ayant une activité d'immunomodulation pour administration par voie parentérale ou orale, consistant en des composés conjugués d'acides aminés ou de polyacides aminés avec une fraction insoluble codée BV 88, qui est isolée à partir de la souche IV-86 ATCC 53966 de Corynebacterium granulosum et qui consiste en peptidoglycane et en glycoprotéines, caractérisé en ce que l'on fait réagir une suspension de la fraction BV 88 activée par un dialdéhyde $CHO-(CH_2)_n-CHO$ dans lequel n est entre 2 et 4, ou conjuguée avec un carbodiimide, avec une acide aminé ou un polyacide aminé pour obtenir la conjugaison.

**8.** Procédé selon la revendication 7, caractérisé en ce que l'activation par le dialdéhyde est effectuée en traitant une suspension à 1-20 mg/ml de BV 88 dans une solution tampon à pH 7,4 par une solution du dialdéhyde dans le même tampon.

**9.** Procédé selon la revendication 7, caractérisé en ce que la réaction pour conjuguer la fraction BV 88 lorsqu'elle est activée par un dialdéhyde est effectuée en traitant une suspension de BV 88 activé contenant entre 1 et 20 mg/ml de BV 88 par une solution d'acide aminé ayant une concentration comprise entre 1 et 20 mg/ml ou par un polyacide aminé sous forme de poudre à un pH de 9,5 à une température comprise entre 4 et 37°C.

**10.** Procédé selon la revendication 7, caractérisé en ce que les acides aminés utilisés pour la conjugaison avec BV 88 activé par un dialdéhyde sont des acides aminés diaminomonocarboxyliques.

**11.** Procédé selon la revendication 7, caractérisé en ce que les polyacides aminés utilisés pour la conjugaison avec BV 88 activé par un dialdéhyde ont un poids moléculaire compris entre 1 000 et 15 000 daltons.

**12.** Procédé selon la revendication 7, caractérisé en ce que le dialdéhyde est le glutaraldéhyde.

**13.** Procédé selon la revendication 7, caractérisé en ce que, lorsque la fraction BV 88 est conjuguée avec un carbodiimide, une solution à 0,1-10 mg/ml d'un acide aminé dans un tampon à pH 5 est ajoutée à une suspension à 1-20 mg/ml de la fraction BV 88 dans le même tampon, et une solution de carbodiimide est ajoutée au mélange obtenu.

**14.** Procédé selon la revendication 7, caractérisé en ce que, lorsque la fraction BV 88 est conjuguée avec un carbodiimide, la réaction de conjugaison de l'acide aminé est effectuée à pH 5 à température ambiante.

**15.** Procédé selon la revendication 7, caractérisé en ce que les acides aminés utilisés pour la conjugaison de BV 88 avec un carbodiimide sont des acides aminés monoamino-monocarboxyliques et monoaminodicarboxyliques.

**16.** Utilisation de dérivés semi-synthétiques consistant en des composés conjugués d'acides aminés ou de polyacides aminés avec une fraction insoluble codée BV 88, qui est isolée à partir de la souche IV-86 ATCC 53966 de Corynebacterium granulosum et qui consiste en peptidoglycane et en glycoprotéines, pour la préparation de compositions pharmaceutiques ayant une activité d'immunomodulation et antitumorale pour administration par voie parentérale ou orale.

**Revendications pour les Etats contractants suivants : GR, ES**

**1.** Procédé de préparation de dérivés semi-synthétiques ayant une activité d'immunomodulation pour administration par voie parentérale ou orale, consistant en des composés conjugués d'acides aminés ou de polyacides aminés avec une fraction insoluble codée BV 88, qui est isolée à partir de la souche IV-86 ATCC 53966 de Corynebacterium granulosum et qui consiste en peptidoglycane et en glycoprotéines, caractérisé en ce que l'on fait réagir une suspension de la fraction BV 88 activée par un dialdéhyde $CHO-(CH_2)_n-CHO$ dans lequel n est entre 2 et 4, ou conjuguée avec un carbodiimide, avec une acide aminé ou un polyacide aminé pour obtenir la conjugaison.

**2.** Procédé selon la revendication 1, caractérisé en ce que l'activation par le dialdéhyde est effectuée en traitant une suspension à 1-20 mg/ml de BV 88 dans une solution tampon à pH 7,4 par une solution du dialdéhyde dans le même tampon.

**3.** Procédé selon la revendication 1, caractérisé en ce que la réaction pour conjuguer la fraction BV 88 lorsqu'elle est activée par un dialdéhyde est effectuée en traitant une suspension de BV 88 activé contenant entre 1 et 20 mg/ml de BV 88 par une solution d'acide aminé ayant une concentration comprise entre 1 et 20 mg/ml ou par un polyacide aminé sous forme de poudre à un pH de 9,5 à une température comprise entre 4 et 37°C.

**4.** Procédé selon la revendication 1, caractérisé en ce que les acides aminés utilisés pour la conjugaison avec BV 88 activé par un dialdéhyde sont des acides aminés diaminomonocarboxyliques.

**5.** Procédé selon la revendication 1, caractérisé en ce que les polyacides aminés utilisés pour la conjugaison avec BV 88 activé par un dialdéhyde ont un poids moléculaire compris entre 1 000 et 15 000 daltons.

**6.** Procédé selon la revendication 1, caractérisé en ce que le dialdéhyde est le glutaraldéhyde.

**7.** Procédé selon la revendication 1, caractérisé en ce que, lorsque la fraction BV 88 est conjuguée avec un carbodiimide, une solution à 0,1-10 mg/ml d'un acide aminé dans un tampon à pH 5 est ajoutée à une suspension à 1-20 mg/ml de la fraction BV 88 dans le même tampon, et une solution de carbodiimide est ajoutée au mélange obtenu.

**8.** Procédé selon la revendication 1, caractérisé en ce que, lorsque la fraction BV 88 est conjuguée avec un carbodiimide, la réaction de conjugaison de l'acide aminé est effectuée à pH 5 à température ambiante.

**9.** Procédé selon la revendication 1, caractérisé en ce que les acides aminés utilisés pour la conjugaison de BV 88 avec un carbodiimide sont des acides aminés monoamino-monocarboxyliques et monoamino-dicarboxyliques.